# EUROPÄISCHE PATENTSCHRIFT

(11) **EP 3 866 694 B1**
(45) Veröffentlichungstag und Bekanntmachung des Hinweises auf die Patenterteilung: **23.11.2022**
(21) Anmeldenummer: 19786755.9
(22) Anmeldetag: 09.10.2019
(51) Int. Cl.: A61B 6/04, G12B 5/00, B25J 9/16, A61N 5/10, A61G 13/00, A61G 13/04, G01B 11/02, G01B 11/16, G01B 21/04

(54) **ANORDNUNG ZUR POSITIONIERUNG UND POSITIONSERFASSUNG EINER VERFORMBAREN LASTTRAGPLATTE**
ASSEMBLY FOR THE POSITIONING AND POSITION DETECTION OF A DEFORMABLE LOAD-BEARING PLATE
ARRANGEMENT DE POSITIONNEMENT ET DE DÉTECTION DE POSITION D'UNE PLAQUE PORTE-CHARGE DÉFORMABLE

(30) Priorität: 19.10.2018 DE 102018126022
(43) Veröffentlichungstag der Anmeldung: 25.08.2021
(73) Patentinhaber: Physik Instrumente (PI) GmbH & Co. KG, 76228 Karlsruhe (DE)
(72) Erfinder: MANKIN, Erik, 76227 Karlsruhe (DE)
(74) Vertreter: Kruspig, Volkmar
(86) Internationale Anmeldenummer: PCT/EP2019/077336
(87) Internationale Veröffentlichungsnummer: WO 2020/078791

(56) Entgegenhaltungen:
- WO-A1-2018/045463
- AT-A1- 502 426
- DE-A1-102017 104 189
- KR-B1- 101 176 414
- US-A1- 2012 076 269
- US-B1- 9 757 658

## Beschreibung

Die Erfindung betrifft eine Anordnung zur Positionierung und Positionserfassung einer verformbaren Lasttragplatte, mit einer Antriebs-Parallelkinematik mit einer Mehrzahl von Antriebs-Beinen, wobei die Lasttragplatte einen Bestandteil der Antriebs-Parallelkinematik bildet oder starr auf einer Plattform derselben fixiert ist.

Sogenannte Parallelkinematiken, insbesondere Hexapoden oder auch Stewart-Plattformen, werden seit geraumer Zeit zur hochgenauen Positionierung von Teilen insbesondere in Produktionsprozessen, aber auch von Messsystemen sowie von Patienten im Kontext bildgebender Diagnoseverfahren oder der Strahlentherapie eingesetzt. Der Einsatzbereich derartiger Anordnungen hat sich in letzter Zeit stark erweitert, und die Genauigkeitsanforderungen sind für eine Reihe von Einsatzgebieten sehr hoch.

Aus der DE 10 2017 104189 ist eine parallelkinematische Vorrichtung bekannt, bei der die Position und die Ausrichtung der Plattform relativ zum Gestell bestimmbar sind, ohne die jeweiligen Längen der Verbindungselemente zwischen Plattform und Gestell, welche einen translatorischen Freiheitsgrad aufweisen, messen zu müssen.

Die WO 1999/028097 A1 offenbart ein Verfahren zur Kalibrierung einer Parallelkinematik, welche auf der Auswertung von Sensordaten hinsichtlich des Abstandes der Ist-Position zu einer Referenzposition der Plattform basiert. Eine Anwendung einer gattungsgemäßen Parallelkinematik zur Positionierung einer Patientenliege eines medizinischen Diagnose- oder Therapiesystems zeigt die US 2018/0110486 A1.

Speziell bei der präzisen Positionierung von Patienten in hochgenauen bildgebenden Diagnosesystemen bzw. in der Strahlentherapie oder bei Schwerlastanwendungen sind Verformungen der Lasttragplatte nicht mehr vernachlässigbar und müssen zur Erreichung der im entsprechenden Kontext bestehenden Genauigkeitsanforderungen berücksichtigt werden.

Der Erfindung liegt daher die Aufgabe zugrunde, eine verbesserte Anordnung zur Positionierung und Positionserfassung einer verformbaren Lasttragplatte anzugeben, mit der hohen Genauigkeitsanforderungen Rechnung getragen werden kann.

Diese Aufgabe wird durch eine Anordnung mit den Merkmalen des Anspruchs 1 gelöst. Zweckmäßige Fortbildungen des Erfindungsgedankens sind Gegenstand der abhängigen Ansprüche.

Ein Gedanke der Erfindung besteht darin, zusätzlich zur Antriebs-Parallelkinematik eine Referenz-Parallelkinematik mit einer durch deren Plattform gebildeten oder auf dieser starr fixierten, im Wesentlichen verformungsfreien Referenzplatte, die unterhalb der Lasttragplatte positioniert ist, und einer Mehrzahl von Referenz-Beinen, bereitzustellen. Die angenommene Verformungsfreiheit der Referenzplatte dieser Referenz-Parallelkinematik wird insbesondere durch eine Ausführung mit hoher Steifigkeit (durch geeignetes Material und/oder geeigneten Innenaufbau und/oder geeignete Geometrie) und dadurch gewährleistet, dass die Referenzplatte weitgehend frei von eingeleiteten Kräften gehalten wird.

Weiterhin ist erfindungsgemäß eine Sensoranordnung vorgesehen, die eine Mehrzahl von in Referenz-Beinen angeordneten Beinlängenmesseinrichtungen sowie eine Mehrzahl von auf der Referenzplatte angeordnete Abstandssensoren zur lokalen (punkt- oder bereichsweisen) Erfassung des Abstandes der Referenzplatte von der Lasttragplatte umfasst. Die Sensoranordnung gewinnt, bezogen auf die nahezu starre Referenzplatte, eine Vielzahl von Positionssignalen zur Beschreibung der räumlichen Lage der (gegebenenfalls durch die getragene Last verformten) Lasttragplatte.

Schließlich umfasst die vorgeschlagene Anordnung eine eingangsseitig mit den Beinlängenmesseinrichtungen und den Abstandssensoren verbundene Positionsberechnungseinheit, die die Ausgangssignale der Beinlängenmesseinrichtungen und Abstandssensoren zu einer Menge dreidimensionlaler Koordinaten von Oberflächenpunkten der Lasttragplatte verarbeitet, wodurch eine Positions-Karte der Lasttragplatte resultiert. Das so gewonnene exakte Abbild der Raumkontur der Lasttragplatte kann Eingangssignale für Korrektureinrichtungen liefern, die beispielsweise die Lasttragplatte umpositionieren oder die lastbedingten Verformungen der Lasttragplatte ggf. unter Ausnutzung einer statischen Überbestimmtheit beim optionalen Einsatz überzähliger bzw. "redundanter" Beine, aktiv kompensieren können. Andererseits können die vorhandenen (und unkompensierten) Verformungen für die Signalauswertung eines zugehörigen bildgebenden Untersuchungssystems oder die Steuerung eines Bearbeitungs- oder Therapiesystems rechnerisch berücksichtigt werden. Z. B. kann eine Anpassung der Strahlführung bei einer Strahlentherapie vorgenommen werden.

In einer Ausführung der Erfindung ist die Referenz-Parallelkinematik als separate Baueinheit zusätzlich zur Antriebs-Parallelkinematik vorgesehen und ihre Plattform als lastfreie Plattform ausgebildet. In dieser Ausführung umfasst die Anordnung Mittel zur lateralen Ausrichtung der Referenzplatte bezüglich der Plattform der Antriebs-Parallelkinematik, wobei die Mittel zur lateralen Ausrichtung insbesondere zusammenwirkende Gleitmittel an der Referenzplatte und der Plattform der Antriebs-Parallelkinematik und eine lateral wirkende Zugfedereinrichtung aufweisen.

In einer alternativen Ausgestaltung ist die Referenz-Parallelkinematik zugleich durch die Antriebs-Parallelkinematik gebildet, wobei die Plattform der Antriebs-Parallelkinematik wesentlich kleiner als die Lasttragplatte und daher angenähert als verformungsfrei anzusehen ist. Hierbei ist die Referenzplatte als zusätzliches Element zur kleinen Plattform ("Gondel") der Antriebs-/Referenz-Parallelkinematik vorgesehen und sowohl mit der Plattform der Parallelkinematik als auch mit der Lasttragplatte verbunden.

In aus derzeitiger Sicht besonders wichtigen und praktisch vielseitig einsetzbaren Ausführung weist die Antriebs-Parallelkinematik und/oder die Referenz-Parallelkinematik einen Hexapoden auf, der mindestens sechs längenverstellbare Beine und als Plattform eine Gondel und insbesondere auch eine Bodenplatte umfasst. Grundsätzlich ist die Erfindung aber auch mit anderen Typen von Parallelkinematiken vorteilhaft einsetzbar und kann auch solche neuen Anwendungsfelder erschließen, in denen hohe Genauigkeitsanforderungen zu erfüllen sind. Während ein Hexapode - wie schon der Name besagt - üblicherweise sechs Beine aufweist, können im Kontext der Erfindung Ausführungen vorteilhaft sein, bei denen die Antriebs-Parallelkinematik mehr als sechs verstellbare Antriebs-Beine umfasst und/oder bei denen die Referenz-Parallelkinematik mehr als sechs Referenz-Beine umfasst, in denen jeweils Beinlängenmesseinrichtungen vorgesehen sind.

Der Einsatz von überzähligen oder "redundanten" Referenz-Beinen ermöglicht eine verfeinerte Auswertung und/oder steuerungsseitige Verwertung der Positionsmessergebnisse, eine verbesserte Fehlerkorrektur bzw. eine technische Fehlererkennung in den Antriebs-Beinen und/oder den Referenz-Beinen, wodurch insgesamt genauere und zuverlässigere Posenmessungen der Referenzplatte und damit eine genauere und zuverlässigere Positionierung der Lasttragplatte resultiert. Unter Pose ist dabei die Kombination von Position und Orientierung eines starren Objektes oder Körpers, z.B. der Referenzplatte, im dreidimensionalen Raum zu verstehen.

Ein Vorsehen "redundanter" Antriebs-Beine erleichtert einerseits das Erkennen von technischer Störungen, falls die Antriebs-Beine mit Längenmesseinrichtungen versehen sind. Andererseits wird hierdurch die Parallelkinematik statisch überbestimmt, so dass neben einer Positionierung der Lasttragplatte auch eine gezielte Verformung derselben bewirkt werden kann. Diese gezielten Verformungen können beispielsweise dem Zweck dienen, lastbedingten Verformungen entgegen zu wirken. Positionierungen und Verformungen der Lasttragplatte können durch die Messungen der Sensoranordnung der Referenz-Parallelkinematik verfolgt und iterativ verfeinert werden. Beim Bewirken von Verformungen durch eine überbestimmte Kinematik empfiehlt sich die Messung der Beinkräfte, zumindest aber die Überwachung der elektrischen Ströme der Bein-Antriebe; darüber hinaus ist es vorteilhaft, wenn die Möglichkeit besteht, "redundante" Beine kraftlos zu schalten.

Bevorzugt sind im Kontext der Erfindung die Beinlängenmesseinrichtungen in den Beinen der Referenz-Parallelkinematik und die Abstandssensoren auf der Referenzplatte als Absolutwert-Sensoren ausgeführt. Grundsätzlich ist auch mit Relativwert-Sensoren eine Implementierung der Erfindung möglich, dies würde jedoch jeweils zusätzliche Kalibrierungsschritte erfordern und die Ausführung aufwendiger machen.

Im Normalfall können die Abstandssensoren an den Eckpunkten mindestens eines gleichseitigen Dreiecks oder Rechtecks, insbesondere in einer regulären Anordnung angeordnet sein, die aus mehreren gleichseitigen Dreiecken oder Rechtecken gebildet ist. Es versteht sich, dass auch andere Vieleck-Anordnungen der Abstandssensoren (etwa an den Punkten eines oder mehrerer Sechseck/e) in Betracht kommen, sofern den Anforderungen des konkreten Anwendungsfalles damit besser Rechnung getragen wird.

In weiteren Ausführungen sind die Abstandssensoren auf der Referenzplatte derart positionsveränderlich angeordnet dass ihre Position in Abstimmung auf mechanische Eigenschaften der Lasttragplatte und optional auf eine spezielle Lastkonfiguration eingestellt werden kann. Dies erfordert zwar eine aufwendigere Konstruktion der Referenzplatte mit entsprechenden verstellbaren Halterungen bzw. Führungen für die Abstandssensoren, ermöglicht aber deren flexiblere Anpassung an vielgestaltige Anwendungssituationen.

In einer wichtigen Anwendung der Anordnung ist die Lasttragplatte als Patientenliege eines medizinischen Diagnose- oder Therapiesystems ausgebildet. In einem weiteren wichtigen Anwendungsfall ist die Lasttragplatte als Schwerlast-Plattform als Teil eines technischen Mess- oder Bearbeitungssystems ausgebildet. Es versteht sich, dass die Erfindung mit besonderem Vorteil auch in weiteren Anwendungsfeldern einsetzbar ist, in denen relativ große und zugleich ausgedehnte Lasten auf einer entsprechend ausgedehnten Lasttragplatte platziert sind.

In einer praktisch bedeutsamen erweiterten Konfiguration hat die Anordnung eine Positionsnachführungseinheit, die eingangsseitig mit einem Ausgang der Positionsberechnungseinheit verbunden ist und die Positionskarte der Lasttragplatte repräsentierende Daten empfängt und anhand dieser Daten eine gegebenenfalls iterative Positionsnachführung der Lasttragplatte in deren belastetem und verformtem Zustand bewirkt.

Im Hinblick auf die weiter oben erwähnte Konfiguration mit "redundanten" oder überzähligen Beinen kann hierbei die Positionsnachführungseinheit mindestens einen Ausgang aufweisen, der mit einem der überzähligen Antriebs-Beine der Antriebs-Parallelkinematik zur Längenverstellung desselben verbunden ist.

Ist eine Parallelkinematik überbestimmt (also beispielsweise ein "Hexapod" mit sieben Beinen), so wird durch die Vorgabe von genau 6 Beinlängen eine Pose definiert. Wird dann noch ein "redundantes" Bein bewegt, kommt es zu einer Verspannung der Gondel. Die auftretenden maximalen Kräfte hängen von den maximal realisierbaren Beinkräften, der Elastizität der Kinematik und letztlich auch von der der mechanischen Belastbarkeit/Stabilität der Kinematik ab. Es können sehr große Bein-Kräfte und überhaupt Spannungen in unbekannter Größe in der Mechanik auftreten, die ernsthafte Folgen haben können. Daher ist es vorteilhaft, in redundanten Beinen Kraftmesseinrichtungen vorzusehen, um solche Zustände rechtzeitig erfassen und ihnen entgegenwirken zu können.

Vorteile und Zweckmäßigkeiten der Erfindung ergeben sich im Übrigen aus der nachfolgenden Beschreibung von Ausführungsbeispielen anhand der Figuren.

Die Figuren zu den Ausführungsbeispielen zeigen:
- Fig. 1: eine Seitenansicht einer durch eine Parallelkinematik angetriebenen Patientenliege in bekannter Bauart,
- Fig. 2A und 2B: eine perspektivische Darstellung sowie eine Seitenansicht einer Patientenliege mit einer erfindungsgemäßen Anordnung zur Positionierung und Positionserfassung,
- Fig. 3: eine perspektivische Darstellung der Grundplatte und der Referenz-Parallelkinematik der Anordnung nach Fig. 2A und 2B,
- Fig. 4A, 4B und 4C: eine perspektivische Untersicht sowie zwei Seitenansichten der Referenzplatte und Lasttragplatte zur Erläuterung der Verbindungsanordnung zwischen diesen,
- Fig. 5: eine perspektivische Darstellung eines weiteren Konstruktionsdetails der Referenzplatte,
- Fig. 6: eine Seitenansicht der Referenz-Parallelkinematik,
- Fig. 7: eine Seitenansicht einer modifizierten Ausführungsform der in Fig. 2A und 2B gezeigten Patientenliege,
- Fig. 8: eine perspektivische Darstellung der Referenzplatte der Patientenliege nach Fig. 7,
- Fig. 9, 10A und 10B: schematische Darstellungen zur Erläuterung der Funktionsweise der Positionssensorik bei einer weiteren beispielhaften Anordnung.

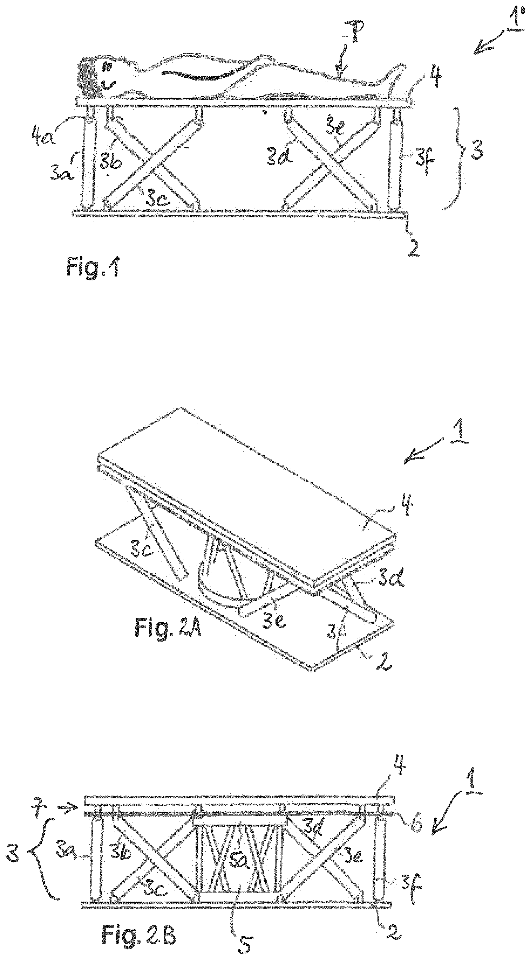

Fig. 1 zeigt den Aufbau einer an sich bekannten Patientenliege 1' für den Einsatz mit bildgebenden Diagnosegeräten, etwa einem Röntgen- oder Computertomographiesystem, oder für den Einsatz in der Strahlentherapie mit einem darauf liegenden Patienten P. Diese Patientenliege stellt eine gattungsgemäße Anordnung dar und umfasst eine Bodenplatte 2 und eine Parallelkinematik 3, zu der die als Patientenunterlage dienende Lasttragplatte 4 sowie sechs Antriebs-Beine 3a bis 3f gehören.

Die Parallelkinematik 3 ist vom Typ des Hexapoden. Die Antriebs-Beine 3a bis 3f sind an beiden Enden über (nicht bezeichnete) Kugelgelenke einerseits mit der Bodenplatte 2 und andererseits mit der Lasttragplatte 4 verbunden. An den Anlenkpunkten der Antriebs-Beine an der Lasttragplatte sind sogenannte Beinschäfte vorgesehen, von denen hier nur einer beispielhaft mit Ziffer 4a bezeichnet ist und deren Aufbau - wie auch der Aufbau des Hexapoden 3 insgesamt - als bekannt vorausgesetzt wird.

Die Lasttragplatte 4 entspricht funktional der sog. Gondel eines herkömmlichen Hexapoden, unterscheidet sich aber von dieser als starrer Körper angesehenen Gondel durch ihre Verformbarkeit und dadurch, dass ihr daraufhin keine sog. Pose zugeordnet und ihre Erstreckung im Raum nicht (wie bei einem starren Körper) mit sechs Posenparametern beschrieben werden kann.

Durch das Gewicht des Patienten kommt es zu einer Verformung des Hexapoden, und zwar primär der Lasttragplatte 4, was zu einer Verschiebung des Patienten, aber auch der erwähnten Anlenkpunkte führt. Aufgrund dessen dreht sich die Lasttragplatte auch im Raum und verschiebt sich in einem kartesischen Koordinatensystem. Diese Verformung und Raum-Drehung wiederum verfälscht aus dem Antrieb der Lasttragplatte gewonnene Positionssignale, die in die Auswertung der durch das bildgebende Diagnosesystem gewonnenen Daten einfließen, und hat somit letztlich eine verringerte Genauigkeit dieser Daten zur Folge.

Fig. 2A und 2B zeigen in einer perspektivischen Draufsicht bzw. Seitenansicht eine Patientenliege 1 als Beispiel einer erfindungsgemäßen Anordnung. Die Anordnung ähnelt der in Fig. 1 dargestellten bekannten Anordnung, und funktional übereinstimmende oder weitgehend übereinstimmende Teile sind mit den gleichen Bezugsziffern wie in Fig. 1 bezeichnet.

Bei der Anordnung 1 ist zusätzlich zur Antriebs-Kinematik 3 eine Referenz-Kinematik 5 mit einer Gondel 5a und aufgesetzter Referenzplatte 6 vorgesehen, welche die mechanische Grundlage für eine hochgenaue, lastbedingte Verformungen berücksichtigende Positionserfassung und darauf beruhenden Positionierung des Patienten darstellt. Die hierzu weiterhin gehörige Sensorik und Auswertetechnik sind in Fig. 2A und 2B nicht dargestellt, und die Beine (nachfolgend: Referenz-Beine) der Referenz-Parallelkinematik 5 sind hier nicht einzeln bezeichnet.

Wie in Fig. 2B zu erkennen ist, sind bei der beispielhaften erfindungsgemäßen Anordnung 1 die Antriebs-Beine 3a bis 3f an der Unterseite der Referenzplatte 6 angelenkt, also nicht wie bei der bekannten Anordnung direkt an der Lasttragplatte 4. Um eine Übertragung der Antriebskraft und entsprechende Positionierung der Lasttragplatte 4 zu gewährleisten, ist zwischen der Referenzplatte 6 und der Lasttragplatte 4 eine speziell gestaltete Verbindungsanordnung 7 vorgesehen, die weiter unten genauer beschrieben wird. Die Referenz-Parallelkinematik 5 (beim gezeigten speziellen Aufbau auch als Referenz-Hexapode zu bezeichnen) ist antriebslos und umfasst in den Referenz-Beinen Absolutlängenmesseinrichtungen (oder zumindest referenzierbare Längenmessrichtungen) und ist daher zu einer absoluten Bestimmung der Pose seiner Gondel 5a und somit der Referenzplatte 6 in der Lage, die auf der Gondel 5a starr fixiert ist.

Die Referenz-Parallelkinematik und Referenzplatte 6 sind derart konstruiert, dass auf die Referenzplatte konstruktionsbedingt nur geringste Kräfte wirken. Im Übrigen kann sie aus einem hochgradig steifen Material bestehen und/oder eine hohe Steifigkeit durch eine geeignete Innenkonstruktion haben. Insgesamt ist also davon auszugehen, dass die Referenzplatte keiner relevanten Verformung unterliegt, und ein leicht-gängiger Aufbau des Referenz-Hexapoden gewährleistet, dass zwischen diesem und der Referenzplatte ein lediglich unbedeutender Kraftaustausch stattfindet.

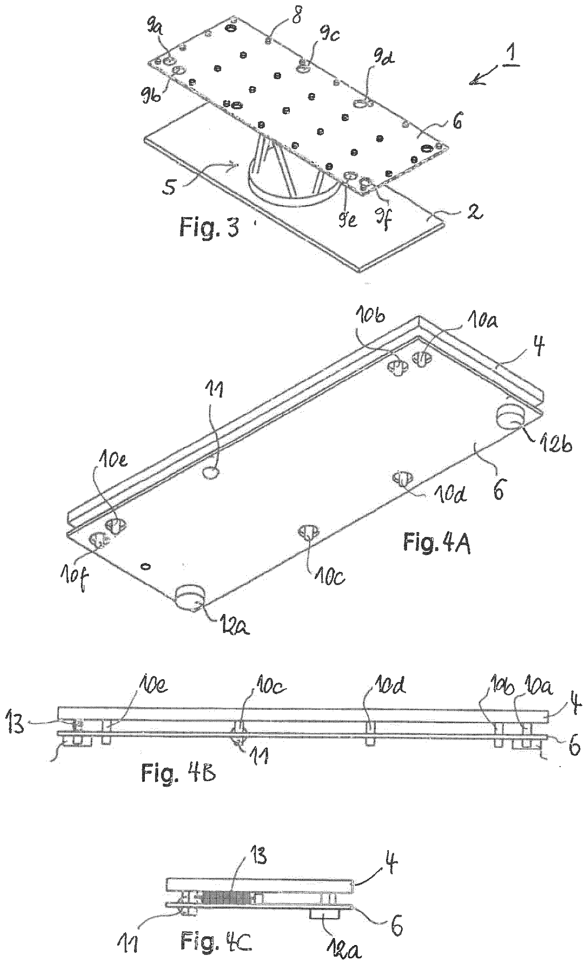

In Fig. 3 ist die auf der Oberseite der Referenzplatte 6 vorgesehene matrixförmige Anordnung von Abstandssensoren 8 zu erkennen, mit denen über die gesamte Fläche der Lasttragplatte deren jeweiliger lokaler Abstand zur Referenzplatte erfasst und damit die Position der Lasttragplatte räumlich aufgelöst erfasst wird. Weiterhin sind in Fig. 3 kreisförmige Öffnungen 9a bis 9f gezeigt, die die nachfolgend beschriebene Funktion haben. Weitere Details der Referenzplatte 6 werden ebenfalls weiter unten erwähnt und erläutert. Auch zur Funktion der Abstands-Sensorik der erfindungsgemäßen Anordnung finden sich weiter unten genauere Erläuterungen.

Die Lasttragplatte ist auf solche Weise mit der Referenzplatte verbunden, dass zwar eine Kraftübertragung zwischen beiden Platten unterbunden, gleichzeitig jedoch die Antriebskraft von den Antriebs-Beinen des Antriebs-Hexapoden 3 in die Lasttragplatte eingeleitet werden kann. Hierzu dienen die in Fig. 3 mit 9a bis 9f bezeichneten, aber auch in Fig. 4A zu erkennenden (jedoch dort nicht gesondert bezeichneten) Durchgangsbohrungen 9a bis 9f der Referenzplatte 9. Wie in Fig. 4A zu erkennen, werden die Durchgangsbohrungen von jeweils einem Beinschaft 10a bis 10f durchdrungen, die die Verbindung zu den (nicht gezeigten) Antriebs-Beinen herstellen. Der Innendurchmesser der Durchgangsöffnungen ist gegenüber dem Außendurchmesser der Beinschäfte derart größer gewählt, dass unter allen denkbaren Betriebsbedingungen keine laterale Kraftübertragung von den Beinschäften auf die Referenzplatte erfolgt.

Um ein reibungsarmes Aufliegen der Lasttragplatte 4 auf der Referenzplatte 6 zu gewährleisten, weist diese ein Kugellager bzw. Kugelgelenk 11 nahe einer der langen Seitenkanten und in einem Mittenbereich derselben sowie zwei Gleitlager 12a, 12 b nahe den Endpunkten der gegenüberliegenden Seitenkante auf. Die Auflagepunkte bilden somit ein großes Dreieck, welches für eine stabile Auflage sorgt, und wegen der dort angeordneten reibungsarmen Lager ist diese Auflage weitgehend reibungsfrei.

Das Kugelgelenk 11 ermöglicht grundsätzlich eine relativ leichte Drehung der Tragplatte 4 bezüglich der Referenzplatte 6. Um einer solchen entgegenzuwirken, ist im Zwischenraum zwischen beiden Platten eine Rückstellfedereinrichtung 13 angebracht. Die Rückstellfedereinrichtung 13 umfasst beispielsweise eine Schraubenfeder, deren eines Ende um einen der Beinschäfte geschlungen ist und deren anderes Ende an einem Stift auf der Oberfläche der Referenzplatte befestigt ist. Eine gleichfalls an der Referenzplatte befestigte Anschlagplatte (nicht gezeigt) liegt an einem der anderen hindurchgehenden Beinschäfte an und begrenzt die Wirkung der Rückstellfedereinrichtung.

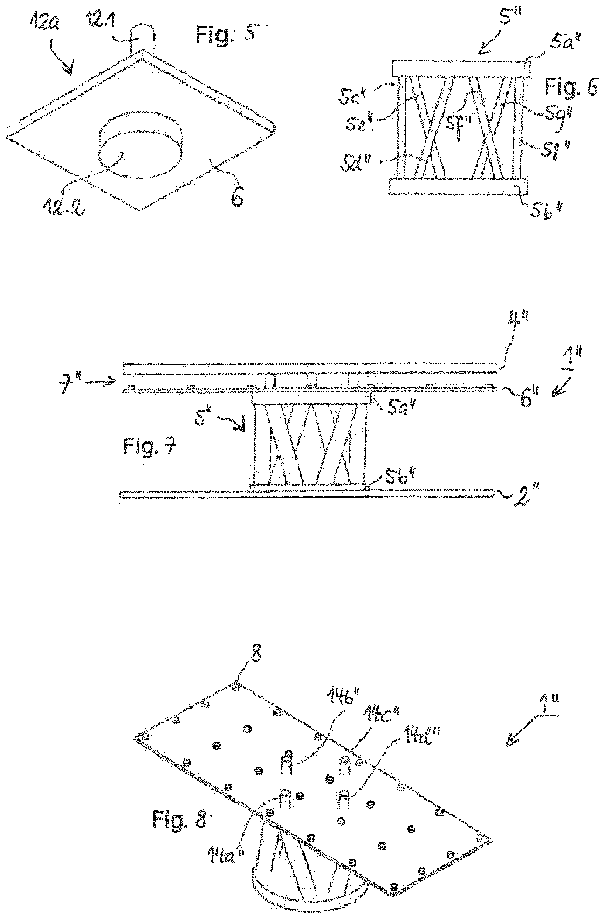

Fig. 5 zeigt schematisch den Aufbau des Gleitlagers 12a, das durch eine an einem Schafft 12.1 angebrachte Kreisscheibe 12.2 gebildet ist, auf deren Oberseite die (hier durch eine kleine quadratische Platte symbolisierte) Referenzplatte 6 aufliegt.

Fig. 6 bis 8 zeigen wesentliche Aspekte einer weiteren erfindungsgemäßen Anordnung 1". Bei dieser Anordnung 1" ist als Referenz-Parallelkinematik ein Hexapode 5" verbaut, der neben der Gondel 5a" auch eine eigene Grundplatte 5b" hat, wie in Fig. 6 separat dargestellt. Dort sind auch die Referenz-Beine mit den Ziffern 5c" bis 5i" bezeichnet. Da der Aufbau der Anordnung 1" ansonsten ähnlich demjenigen der Anordnung 1 aus Fig. 2A bis 3 ist, sind zur Bezeichnung der einzelnen Teile Bezugsziffern vergeben, die an das erste Ausführungsbeispiel angelehnt sind. Gänzlich unverändert sind grundsätzlich nur die Abstandssensoren 8.

Während beim obigen ersten Ausführungsbeispiel das Antriebssystem und das Raumlagen- bzw. Positionsmesssystem, also die Antriebs-Parallelkinematik mit Lasttragplatte einerseits und die Referenz-Parallelkinematik mit Referenzplatte andererseits, mechanisch getrennt sind, ist diese Trennung bei der Anordnung nach Fig.6 bis 8 nicht mehr gegeben, wodurch sich eine einfachere und kostengünstigere Konstruktion ergibt.

Der Hexapode 5" dient hier sowohl zum Antrieb der Lasttragplatte als auch zur räumlich aufgelösten Messung der tatsächlichen Raumlage ihrer einzelnen Punkte bzw. Bereiche. Die Beine 5c" bis 5f" sind also sowohl mit Längenmesseinrichtungen als auch mit Antrieben (nicht gezeigt) bestückt. Die Gondel 5a" ist hier mit der Lasttragplatte 4" fest verbunden, wozu die in Fig. 8 zu erkennenden vier Verbindungsstifte 14a" bis 14d" auf der (ihrerseits mit der Gondel 5a" fest verbundenen) Referenzplatte 6" vorgesehen sind.

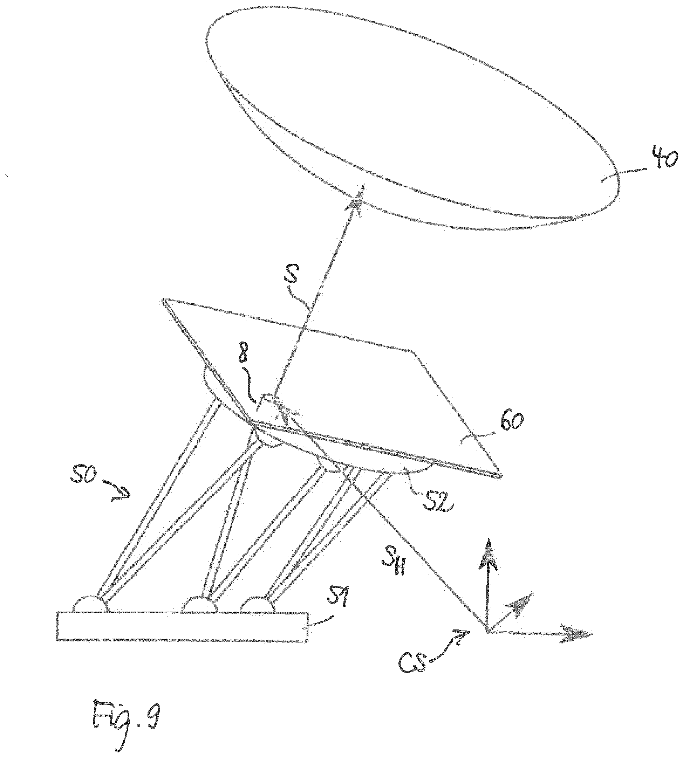

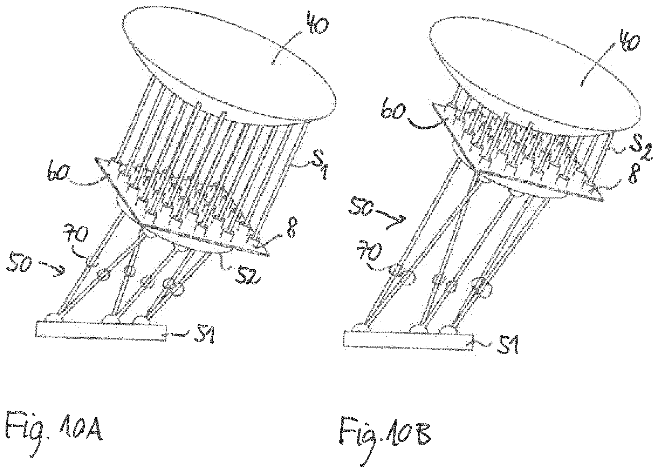

Fig. 9, 10A und 10B sind schematisch perspektivische Darstellungen zur Erläuterung des Funktionsprinzips der (absoluten) Abstandsmessung zwischen Referenzplatte und Lasttragplatte bei den weiter oben beschriebenen Anordnungen. Zur Unterscheidung von jenen konkreten Ausführungsbeispielen sind für die schematischen Darstellungen andere Bezugsziffern gewählt, die gleichwohl an die Ziffern bei den beispielhaften Ausführungen angelehnt sind.

Als Antriebs-/Mess-Parallelkinematik ist hier wieder ein Hexapode 50 gezeigt, dessen Grundplatte mit 51 und dessen Gondel mit 52 bezeichnet ist. Die Referenzplatte ist hier mit Ziffer 60 bezeichnet, oberhalb derselben ist ein allgemeiner Körper 40 in der Gestalt eines flachen Kugelsegments gezeichnet. Dessen untere Oberfläche wird, wie in Fig. 9 mit dem Pfeil S bezeichnet, durch einen Abstandssensor 8 mit einem eng begrenzten Messstrahl beaufschlagt.

Die Richtung des Pfeiles S ist parallel zur Flächennormalen der Referenzplatte 60, und die Länge des Pfeils (Vektors) S repräsentiert den vom Abstandsensor 8 gemessenen Absolutwert. Der Fußpunkt des Vektors S ist der Montagepunkt des Abstandssensors 8, und dessen Koordinaten bezüglich des Referenz-exapoden-Koordinatensystems CS sind bekannt, da sie sich aus der Pose des Hexapoden ergeben. Aus dieser ergibt sich auch die Flächennormale der Referenzplatte, also die Richtung des Vektors S. Die kartesischen, räumlichen Koordinaten des Endpunktes des Vektors S sind durch die Vektorsumme des Vektors S und des Vektors SH zwischen dem Ursprung des Referenzhexapoden-Koordinatensystems CS und dem Fußpunkt des Abstandssensors 8 gegeben.

Ein wesentliches Ergebnis dieses Messvorgangs besteht in der Ermittlung der räumlichen Koordinaten einzelner Oberflächenpunkte der Lasttragplatte im Koordinatensystem des Referenz-Hexapoden CS. Die Menge der so identifizierten räumlichen (dreidimensionalen) Koordinaten von Oberflächenpunkten im Koordinatensystem CS, d.h. die Positions-Karte, beschreibt die Lage der Lasttragplatte im Raum. Aufgrund einer Verbindungsanordnung entsprechend Ausführungsbeispiel 1 bzw. der Konstruktion entsprechend Ausführungsbeispiel 2 sind Referenzplatte und Lasttragplatte gegeneinander im Wesentlichen nur parallel und gleichsinnig verschoben, so dass zusätzlich eine Zuordnung von Oberflächenpunkten von jeweils Referenzplatte und Lasttragplatte gegeben ist.

Ausdrücklich sei gesagt, dass die Pose der Referenzplatte zur Berechnung der genannten Koordinaten benötigt wird, jedoch keine Eignung zur Beschreibung der Lage der Lasttragplatte im Raum hat. Es ist ein Gedanke der Erfindung, dass bei jeder möglichen Klasse von Verbindungsanordnungen von Lasttragplatte und Referenzplatte entsprechend Ausführungsbeispiel 1 Verformungen der Lasttragplatte zu Posenänderungen der Referenzplatte führen, so dass die Pose der Referenzplatte selbst als untergeordnet anzusehen ist und daher nur einen groben Aufschluss über die Lage der Lasttragplatte im Raum geben kann. Es ist ein weiterer Gedanke der Erfindung, dass die Pose der Referenzplatte bei einer Anordnung entsprechend des Ausführungsbeispiels 2 im Wesentlichen die Lage und Orientierung eines begrenzten Flächenstücks der Lasttagfläche widerspiegelt und daher ebenfalls nur einen groben Aufschluss über die Lage der Lasttragplatte im Raum geben kann.

In den Ausführungsbeispielen wird davon ausgegangen, dass die optionale Bodenplatte des Referenzhexapoden bzw. der Referenz-Parallelkinematik auf einem unnachgiebigen und starren Fundament ruht, selbst also keiner Verformung unterworfen ist, und die unteren Koordinaten der Referenzbein-Gelenkpunkte somit keine Verschiebungen erfahren, da andernfalls kein festes Bezugs-Koordinatensystem CS existiert. Diese Bedingung ist beispielsweise erfüllt, wenn die Bodenplatte des Lasthexapoden in dieser Weise gegründet ist und die Bodenplatte des Referenzhexapoden auf dieser verankert ist.

Fig. 10A und 10B zeigen exemplarisch die in Fig. 9 dargestellte Anordnung in zwei verschiedenen Positionen der Referenzplatte 60, einmal mit relativ weit eingefahrenen Beinen des Hexapoden 50 (Fig. 10A) und das andere Mal mit relativ weit ausgefahrenen Beinen, wobei hier die Referenzplatte in Richtung ihrer Flächennormalen bewegt wurde. In diesen Figuren sind alle Abstandssensoren 8 auf der Referenzplatte 60 gezeigt, und einer ihrer Messstrahlen ist beispielhaft mit S₁ (Fig. 10A) bzw. S₂ (Fig. 10B) bezeichnet.

Aufgrund der in Fig. 9 skizzierten und weiter oben erläuterten Absolutmessung werden in beiden Lagen der Referenzplatte dieselben Koordinaten der entsprechenden Oberflächenpunkte der unteren Oberfläche des Körpers 40 im Koordinatensystem CS ermittelt, obgleich die jeweiligen Abstände zwischen der Referenzplatte 60 und dem Körper 40 gravierend unterschiedlich sind. Hieraus wiederum ergibt sich, dass die konkrete Lage der Referenzplatte (die Veränderungen durch wechselnde Belastungen der Lasttragplatte unterliegt) im Falle einer Parallelverschiebung zu ihrer Flächennormalen allenfalls einen vernachlässigbaren Einfluss auf die Genauigkeit der von der Lasttragplatte der Anordnung gewonnenen Positions-Karte hat - vorausgesetzt, dass die Genauigkeit der in den Hexapoden-Beinen untergebrachten Beinlängenmesseinrichtungen mit derjenigen der Abstandssensoren weitgehend vergleichbar ist.

Im Falle einer zusätzlichen - in der Praxis stets geringfügigen und wegen des geringen Abstandes von Referenzplatte und Lasttragplatte nicht ins Gewicht fallenden - Verkippung der Referenzplatte würden Koordinaten anderer Oberflächenpunkte vermessen werden, welche zu den ursprünglichen Oberflächenpunkten geringfügig lateral verschoben sind. Es gilt jedoch auch hier, dass die räumlichen Koordinaten dieser neuen Oberflächenpunkte im Koordinatensystem CS korrekt bestimmt werden.

In Fig. 10A und 10B sind symbolisch auch Längenmesseinrichtungen 70 in den Beinen des Hexapoden 50 dargestellt, die bereits weiter oben erwähnt wurden. Diese Längenmesseinrichtungen in Hexapoden-Beinen sind an sich bekannt und werden daher hier nicht weiter beschrieben. Neben den Abstandssensoren 8 liefern sie den für die räumlich aufgelöste Positionsbestimmung des Körpers 40 (bzw. in der Praxis der Lasttragplatte) benutzten Daten.

Im Sinne der obigen Erläuterungen zu Fig. 9 sind dies Daten, die in die Bestimmung des Endpunktes des Vektors SH und damit letztlich in die Vektorsumme der Vektoren S und SH einfließen. Es ist leicht einzusehen, dass die Beinlängen-Daten bei den in Fig. 10A und 10B beispielhaft gezeigten Posen und somit auch die entsprechenden Vektoren SH zu den Fußpunkten der einzelnen Abstandssensoren unterschiedlich sind. Die Vektoraddition mit den jeweils ebenfalls unterschiedlichen Abstands-Vektoren S ergibt aber für beide Figuren übereinstimmende Vektorsummen.

Die Ausführung der Erfindung ist nicht auf die oben beschriebenen Beispiele und hervorgehobenen Aspekte beschränkt, sondern ebenso eine Vielzahl von Abwandlungen möglich, wenn diese im Schutzbereich der anhängenden Ansprüche und im Rahmen fachgemäßen Handelns liegen.

## Patentansprüche

1. Anordnung (1;1") zur Positionierung und Positionserfassung einer verformbaren Lasttragplatte, aufweisend:
eine Antriebs-Parallelkinematik (3;5";50) mit einer Mehrzahl von Antriebs-Beinen, wobei die Lasttragplatte (4;4";40) einen Bestandteil der Antriebs-Parallelkinematik bildet oder starr auf einer Plattform derselben fixiert ist,
**gekennzeichnet durch**
eine Referenz-Parallelkinematik (5;5";50) mit einer durch deren Plattform (5a;5a";52) gebildeten oder auf dieser starr fixierten, im Wesentlichen verformungsfreien Referenzplatte (6;6";60), die unterhalb der Lasttragplatte positioniert ist, und einer Mehrzahl von Referenz-Beinen,
eine Sensoranordnung (8;70), die eine Mehrzahl von in den Referenz-Beinen angeordneten Beinlängenmesseinrichtungen (70) sowie eine Mehrzahl von auf der Referenzplatte angeordnete Abstandssensoren (8) zur punkt- oder bereichsweisen Erfassung des Abstandes der Referenzplatte von der Lasttragplatte umfasst, und
eine eingangsseitig mit den Beinlängenmesseinrichtungen und den Abstandssensoren verbundene Positionsberechnungseinheit, die die Ausgangssignale der Beinlängenmesseinrichtungen und Abstandssensoren zu einer Positions-Karte der Lasttragplatte verarbeitet.

2. Anordnung nach Anspruch 1, wobei die Referenz-Parallelkinematik als separate Baueinheit (5) zusätzlich zur Antriebs-Parallelkinematik (3) vorgesehen und ihre Plattform (5a) als lastfreie Plattform ausgebildet ist.

3. Anordnung nach Anspruch 2, mit Mitteln zur lateralen Ausrichtung der Referenzplatte bezüglich der Plattform der Antriebs-Parallelkinematik, wobei die Mittel zur lateralen Ausrichtung insbesondere zusammenwirkende Gleitmittel an der Referenzplatte und der Plattform der Antriebs-Parallelkinematik und eine lateral wirkende Zugfedereinrichtung umfassen.

4. Anordnung nach Anspruch 1, wobei die Referenz-Parallelkinematik (5") zugleich durch die Antriebs-Parallelkinematik gebildet ist, wobei die Plattform (5a") der Antriebs-Parallelkinematik wesentlich kleiner als die Lasttragplatte und daher angenähert als verformungsfrei anzusehen ist.

5. Anordnung nach Anspruch 4, wobei die Referenzplatte (6") als zusätzliches Element zur Plattform (5a") der Antriebs-/Referenz-Parallelkinematik (5") vorgesehen und sowohl mit der Plattform der Parallelkinematik als auch mit der Lasttragplatte (4") fest verbunden ist.

6. Anordnung nach einem der vorangehenden Ansprüche, wobei die Antriebs-Parallelkinematik (3;5";50) und/oder die Referenz-Parallelkinematik (5;5";50) einen Hexapoden aufweist, der mindestens sechs längenverstellbare Beine und als Plattform eine Gondel (5a;5a";52) und insbesondere auch eine Bodenplatte (2;2";51) umfasst.

7. Anordnung nach Anspruch 6, wobei die Antriebs-Parallelkinematik mehr als sechs verstellbare Antriebs-Beine umfasst, wobei jedes der mehr als sechs Antriebs-Beine ein redundantes Antriebs-Bein sein kann.

8. Anordnung nach Anspruch 6 oder 7, wobei die Referenz-Parallelkinematik mehr als sechs Referenz-Beine umfasst, in denen jeweils Beinlängenmesseinrichtungen vorgesehen sind.

9. Anordnung nach Anspruch 7, wobei wenigstens ein redundantes Antriebs-Bein der Antriebs-Parallelkinematik eine Kraftmesseinrichtung aufweist.

10. Anordnung nach einem der vorangehenden Ansprüche, wobei die Beinlängenmesseinrichtungen (70) in den Beinen der Referenz-Parallelkinematik (5;5";50) und die Abstandssensoren (8) auf der Referenzplatte (6;6";60) als Absolutwert-Sensoren ausgeführt sind.

11. Anordnung nach einem der vorangehenden Ansprüche, wobei die Abstandssensoren (8) an den Eckpunkten mindestens eines gleichseitigen Dreiecks oder Rechtecks, insbesondere in einer regulären Anordnung angeordnet sind, die aus mehreren gleichseitigen Dreiecken oder Rechtecken gebildet ist.

12. Anordnung nach einem der vorangehenden Ansprüche, wobei die Abstandssensoren (8) auf der Referenzplatte (6;6";60) positionsveränderlich angeordnet sind, derart, dass ihre Position in Abstimmung auf mechanische Eigenschaften der Lasttragplatte (4;4";40) und optional auf eine spezielle Lastkonfiguration eingestellt werden kann.

13. Anordnung nach einem der vorangehenden Ansprüche, wobei die Lasttragplatte (4;4";40) als Patientenliege eines medizinischen Diagnose- oder Therapiesystems ausgebildet ist.

14. Anordnung nach einem der vorangehenden Ansprüche, wobei die Lasttragplatte (4;4";40) als Schwerlast-Plattform als Teil eines technischen Mess- oder Bearbeitungssystems ausgebildet ist.

15. Anordnung nach einem der vorangehenden Ansprüche, mit einer Positionsnachführungseinheit, die eingangsseitig mit einem Ausgang der Positionsberechnungseinheit verbunden ist und die Positionskarte der Lasttragplatte repräsentierende Daten empfängt und anhand dieser Daten eine Positionsnachführung der Lasttragplatte in deren belastetem und verformtem Zustand oder eines Bearbeitungs-, Diagnose- oder Therapiewerkzeuges einer Bearbeitungs-, Diagnose- oder Therapieanordnung bewirkt.

16. Anordnung nach Anspruch 15, wobei die Positionsnachführungseinheit mindestens einen Ausgang aufweist, der mit wenigstens einem der Antriebs-Beine der Antriebs-Parallelkinematik zur Längenverstellung desselben verbunden ist.

## Claims

1. An assembly (1; 1") for the positioning and position detection of a deformable load-bearing plate, including:
a drive parallel-type cinematic system (3; 5"; 50) having a plurality of drive legs, wherein the load-bearing plate (4; 4"; 40) forms a component of the drive parallel-type cinematic system or is rigidly fixed on a platform of it,
**characterized by**
a reference parallel-type cinematic system (5; 5", 50) having a substantially deformation-free reference plate (6; 6"; 60) formed by the platform (5a; 5a", 52) thereof or rigidly fixed on it, which is positioned below the load-bearing plate, and having a plurality of reference legs, a sensor arrangement (8; 70) comprising a plurality of leg length measurement devices (70) arranged within the reference legs, as well as a plurality of distance sensors (8) arranged on the reference plate for the point-wise or range-based detection of the distance of the reference plate from the load-bearing plate, and
a position calculation unit connected to the leg length measurement devices and the distance sensors on an input side, which processes the output signals of the leg length measurement devices and the distance sensors to a position map of the load-bearing plate.

2. The assembly according to claim 1, wherein the reference parallel-type cinematic system is provided as a separate assembly unit (5) in addition to the drive parallel-type cinematic system (3), and its platform (5a) is formed as a load-free platform.

3. The assembly according to claim 2, having means for the lateral orientation of the reference plate relative to the platform of the drive parallel-type cinematic system, wherein the means for the lateral orientation in particular comprises cooperating sliding means on the reference plate and the platform of the drive parallel-type cinematic system, and a laterally acting tension spring device.

4. The assembly according to claim 1, wherein the reference parallel-type cinematic system (5") is at the same time formed by the drive parallel-type cinematic system, wherein the platform (5a") of the drive parallel-type cinematic system is considerably smaller than the load-bearing plate and therefor has to be considered approximatively as being deformation-free.

5. The assembly according to claim 4, wherein the reference plate (6") is provided as an additional element to the platform (5a") of the drive/reference parallel-type cinematic system (5") and is fixedly connected both to the platform of the parallel-type cinematic system and the load-bearing plate (4").

6. The assembly according to any one of the preceding claims, wherein the drive parallel-type cinematic system (3; 5"; 50) and/or the reference parallel-type cinematic system (5; 5", 50) include/s a hexapod comprising at least six length-adjustable legs, and, as a platform, a nacelle (5a; 5a"; 52), and in particular also a bottom plate (2; 2"; 51).

7. The assembly according to claim 6, wherein the drive parallel-type cinematic system comprises more than six adjustable drive legs, wherein each of the more than six drive legs may be a redundant drive leg.

8. The assembly according to claim 6 or 7, wherein the reference parallel-type cinematic system comprises more than six reference legs in each of which leg length measurement devices are provided.

9. The assembly according to claim 7, wherein at least one redundant drive leg of the drive parallel-type cinematic system includes a force measurement device.

10. The assembly according to any one of the preceding claims, wherein the leg length measurement devices (70) in the legs of the reference parallel-type cinematic system (5; 5", 50) and the distance sensors (8) on the reference plate (6; 6"; 60) are implemented as absolute value sensors.

11. The assembly according to any one of the preceding claims, wherein the distance sensors (8) are arranged at the corner points of at least one equilateral triangle or rectangle, in particular in a regular arrangement formed of several equilateral triangles or rectangles.

12. The assembly according to any one of the preceding claims, wherein the distance sensor (8) are arranged on the reference plate (6; 6"; 60) to be positionally variable such that the position thereof is adjustable in coordination with mechanical properties of the load-bearing plate (4; 4"; 40) and optionally with a special load configuration.

13. The assembly according to any one of the preceding claims, wherein the load-bearing plate (4; 4"; 40) is formed as a patient couch of a medical diagnostic or therapeutic system.

14. The assembly according to any one of the preceding claims, wherein the load-bearing plate (4; 4"; 40) is formed as heavy-load platform as a part of a technical measurement or processing system.

15. The assembly according to any one of the preceding claims, having a position tracking unit connected to an output of the position calculation unit at an input side and receiving data representing the position map of the load-bearing plate, and causes by means of these data a position tracking of the load-bearing plate in its loaded and deformed state, or of a processing, diagnostic or therapeutic tool of a processing, diagnostic or therapeutic assembly.

16. The assembly according to claim 15, wherein the position tracking unit includes at least one output connected to at least one of the drive legs of the drive parallel-type cinematic system for the length adjustment thereof.

## Revendications

1. Arrangement (1 ; 1") de positionnement et de détection de position d'une plaque porte-charge déformable, comprenant :
une cinématique parallèle d'entraînement (3 ; 5" ; 50) présentant une pluralité de pieds d'entraînement, la plaque porte-charge (4 ; 4" ; 40) faisant partie intégrante de la cinématique parallèle d'entraînement ou étant fixée rigidement sur une plate-forme de celle-ci,
**caractérisé par**
une cinématique parallèle de référence (5 ; 5" ; 50) présentant une plaque de référence (6 ; 6" ; 60) sensiblement indéformable, formée par sa plate-forme (5a ; 5a" ; 52) ou fixée rigidement sur celle-ci, qui est positionnée en dessous de la plaque porte-charge, et une pluralité de pieds de référence,
un ensemble capteur (8 ; 70) présentant une pluralité de dispositifs de mesure de longueur de pied (70) disposés dans les pieds de référence, ainsi qu'une pluralité de capteurs de distance (8) disposés sur la plaque de référence pour la détection ponctuelle ou locale de la distance de la plaque de référence par rapport à la plaque porte-charge, et
une unité de calcul de position qui est connectée, du côté entrée, aux dispositifs de mesure de longueur de pied et aux capteurs de distance et qui traite les signaux de sortie des dispositifs de mesure de longueur de pied et des capteurs de distance pour donner une carte de position de la plaque porte-charge.

2. Arrangement selon la revendication 1,
dans lequel la cinématique parallèle de référence est prévue en tant qu'unité de construction séparée (5) en plus de la cinématique parallèle d'entraînement (3), et sa plate-forme (5a) est réalisée sous forme de plate-forme sans charge.

3. Arrangement selon la revendication 2, comprenant des moyens d'orientation latérale de la plaque de référence par rapport à la plate-forme de la cinématique parallèle d'entraînement, les moyens d'orientation latérale comprenant en particulier des moyens de glissement coopérants sur la plaque de référence et sur la plate-forme de la cinématique parallèle d'entraînement et un dispositif à ressort de traction agissant latéralement.

4. Arrangement selon la revendication 1,
dans lequel la cinématique parallèle de référence (5") est formée en même temps par la cinématique parallèle d'entraînement, la plate-forme (5a") de la cinématique parallèle d'entraînement étant sensiblement plus petite que la plaque porte-charge et pouvant donc être considérée comme étant approximativement indéformable.

5. Arrangement selon la revendication 4,
dans lequel la plaque de référence (6") est prévue comme élément supplémentaire à la plate-forme (5a") de la cinématique parallèle d'entraînement/de référence (5") et est solidaire aussi bien de la plate-forme de la cinématique parallèle que de la plaque porte-charge (4").

6. Arrangement selon l'une des revendications précédentes,
dans lequel la cinématique parallèle d'entraînement (3 ; 5" ; 50) et/ou la cinématique parallèle de référence (5 ; 5" ; 50) comporte un hexapode qui comprend au moins six pieds réglables en longueur et qui présente, en tant que plate-forme, une nacelle (5a ; 5a" ; 52) et en particulier également une plaque de fond (2 ; 2" ; 51).

7. Arrangement selon la revendication 6,
dans lequel la cinématique parallèle d'entraînement comprend plus de six pieds d'entraînement réglables, chacun desdits plus de six pieds d'entraînement pouvant être un pied d'entraînement redondant.

8. Arrangement selon la revendication 6 ou 7,
dans lequel la cinématique parallèle de référence comprend plus de six pieds de référence, dans chacun desquels sont prévus des dispositifs de mesure de longueur de pied.

9. Arrangement selon la revendication 7,
dans lequel au moins un pied d'entraînement redondant de la cinématique parallèle d'entraînement comprend un dispositif de mesure de force.

10. Arrangement selon l'une des revendications précédentes,
dans lequel les dispositifs de mesure de longueur de pied (70) dans les pieds de la cinématique parallèle de référence (5 ; 5" ; 50) et les capteurs de distance (8) sur la plaque de référence (6 ; 6" ; 60) sont réalisés sous forme de capteurs de valeur absolue.

11. Arrangement selon l'une des revendications précédentes,
dans lequel les capteurs de distance (8) sont disposés aux sommets d'au moins un triangle équilatéral ou rectangle, en particulier selon une disposition régulière qui est constituée de plusieurs triangles équilatéraux ou rectangles.

12. Arrangement selon l'une des revendications précédentes,
dans lequel les capteurs de distance (8) sur la plaque de référence (6 ; 6" ; 60) sont disposés de manière à pouvoir changer de position, de sorte que leur position peut être réglée en fonction des propriétés mécaniques de la plaque porte-charge (4 ; 4" ; 40) et, en option, en fonction d'une configuration de charge particulière.

13. Arrangement selon l'une des revendications précédentes,
dans lequel la plaque porte-charge (4 ; 4" ; 40) est réalisée sous forme de lit de patient d'un système médical de diagnostic ou de thérapie.

14. Arrangement selon l'une des revendications précédentes,
dans lequel la plaque porte-charge (4 ; 4" ; 40) est réalisée sous forme de plate-forme pour charges lourdes faisant partie d'un système technique de mesure ou d'usinage.

15. Arrangement selon l'une des revendications précédentes, comportant une unité de suivi de position qui est connectée, du côté entrée, à une sortie de l'unité de calcul de position et qui reçoit des données représentant la carte de position de la plaque porte-charge et qui, à l'aide de ces données, provoque un suivi de position de la plaque porte-charge, dans son état chargé et déformé, ou d'un outil d'usinage, de diagnostic ou de thérapie d'un ensemble d'usinage, de diagnostic ou de thérapie.

16. Arrangement selon la revendication 15,
dans lequel l'unité de suivi de position présente au moins une sortie connectée à l'un au moins des pieds d'entraînement de la cinématique parallèle d'entraînement pour le réglage en longueur dudit pied.
